# EUROPEAN PATENT APPLICATION

(11) **EP 4 306 680 A1**
(43) Date of publication of application: **17.01.2024**
(21) Application number: 22767019.7
(22) Date of filing: 04.03.2022
(51) Int. Cl.: C25B 3/03, C10L 3/08, C25B 1/04, C25B 1/23, C25B 9/00, C25B 15/023

(54) **METHANE PRODUCTION SYSTEM**

(30) Priority: 11.03.2021 JP 2021039696
(71) Applicant: NGK Insulators, Ltd., Nagoya-shi, Aichi 467-8530 (JP)
(72) Inventor: KAN, Hirofumi, Nagoya-shi, Aichi 467-8530 (JP); TORII, Atsushi, Nagoya-shi, Aichi 467-8530 (JP)
(74) Representative: MERH-IP Matias Erny Reichl Hoffmann Patentanwälte PartG mbB
(86) International application number: PCT/JP2022/009367
(87) International publication number: WO 2022/191061

(57) **Abstract**

A methane production system (1) includes a co-electrolysis device (30), and a reforming device (40) connected to the co-electrolysis device (30). The co-electrolysis device (30) has a co-electrolysis cell (32) including a hydrogen electrode (2) at which H₂, CO, and O²⁻ are produced from COz and HzO, an electrolyte (3) capable of transferring O²⁻, and an oxygen electrode (4) at which Oz is produced from the O²⁻ transferred from the hydrogen electrode (2) through the electrolyte (3). The reforming device (40) produces CH₄ from the H₂ and CO produced at the hydrogen electrode (2).

## Description

### TECHNICAL FIELD

The present invention relates to a methane production system.

### BACKGROUND ART

Patent Literature 1 discloses a solid oxide electrolysis cell (abbreviated as "SOEC" hereinafter) provided with a hydrogen electrode at which H₂O is electrolyzed, an electrolyte capable of transferring O²⁻, and an oxygen electrode at which O₂ is produced from O²⁻ transferred from the hydrogen electrode through the electrolyte.

Patent Literature 2 discloses that H₂ and CO can be produced by co-electrolyzing COz and H₂O at the hydrogen electrode of an SOEC.

### CITATION LIST

### Patent Literature

Patent Literature 1: JP 2018-154864A
Patent Literature 2: JP 2019-175636A

### SUMMARY

### TECHNICAL PROBLEM

In order to utilize, as fuel, H₂ and CO produced from COz and H₂O through co-electrolysis using an SOEC, it is effective to produce CH₄ from H₂ and CO using a reforming device.

However, in order to transport H₂ and CO from a plant where an SOEC is installed to a plant where a reforming device is installed, H₂ and CO need to be separated and liquefied or compressed to gas.

Therefore, there is demand for performing production of H₂ and CO and production of CH₄ on-site (i.e., in one facility).

The present invention has been made in view of the above-described circumstances, and aims to provide a methane production system capable of performing production of Hz and CO and production of CH₄ on-site.

### SOLUTION TO PROBLEM

A methane production system according to the present invention includes a co-electrolysis device and a reforming device connected to the co-electrolysis device. The co-electrolysis device has a co-electrolysis cell including a first electrode at which H₂, CO, and O²⁻ are produced from COz and HzO, an electrolyte capable of transferring O²⁻, and a second electrode at which Oz is produced from the O²⁻ transferred from the first electrode through the electrolyte. The reforming device has a reforming cell that produces CH₄ from the H₂ and CO produced at the first electrode.

### ADVANTAGEOUS EFFECTS

According to the present invention, it is possible to provide a methane production system capable of performing production of H₂ and CO and production of CH₄ on-site.

### BRIEF DESCRIPTION OF DRAWINGS

FIG. 1 is a block diagram showing a configuration of a methane production system.
FIG. 2 is a perspective view of a co-electrolysis device.
FIG. 3 is a cross-sectional view of the co-electrolysis device.
FIG. 4 is a perspective view of a co-electrolysis cell.
FIG. 5 is a cross-sectional view of the co-electrolysis cell.
FIG. 6 is a perspective view of a reforming device.
FIG. 7 is a cross-sectional view of the reforming device.
FIG. 8 is a perspective view of a reforming cell.
FIG. 9 is a cross-sectional view of the reforming cell.
FIG. 10 is a block diagram showing another configuration of a methane production system.

### DESCRIPTION OF EMBODIMENTS

### Methane production system

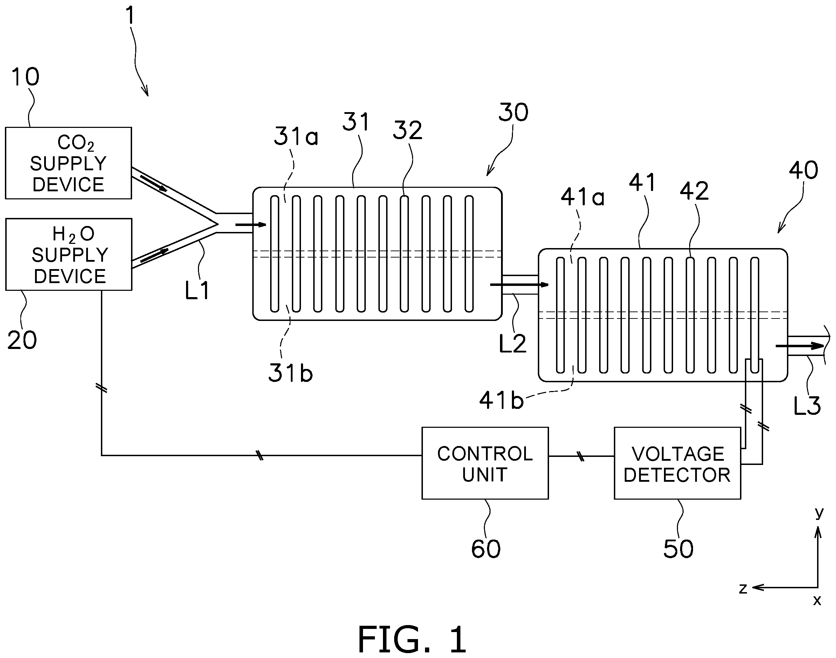

FIG. 1 is a block diagram showing a configuration of a methane production system 1 according to this embodiment.

The methane production system 1 includes a COz supply device 10, an H₂O supply device 20, a co-electrolysis device 30, a reforming device 40, a voltage detector 50, and a control unit 60.

The COz supply device 10 is connected to the co-electrolysis device 30 via a first pipe L1. The COz supply device 10 supplies COz (carbon dioxide) to the co-electrolysis device 30. It is preferable that the amount of CO₂ supplied from the COz supply device 10 to the co-electrolysis device 30 is constant during operation of the co-electrolysis device 30. Accordingly, it is possible to suppress the production of C (solid carbon) and COz due to disproportionation reaction of CO (carbon monoxide) produced in the co-electrolysis cells 32 of the co-electrolysis device 30. As a result, it is possible to suppress deterioration of the electrode activity of the hydrogen electrode 2 of each co-electrolysis cell 32, which will be described later.

The H₂O supply device 20 is connected to the co-electrolysis device 30 via the first pipe L1. The H₂O supply device 20 supplies H₂O (water content) to the co-electrolysis device 30. The entirety or most of H₂O supplied from the H₂O supply device 20 to the co-electrolysis device 30 is gas (steam), but part of the H₂O may be liquid (water). H₂O supplied from the H₂O supply device 20 to the co-electrolysis device 30 may be vaporized using heat of methane (CH₄)-containing gas flowing through the later-described third pipe L3. During operation of the co-electrolysis device 30, the amount of H₂O supplied from the H₂O supply device 20 to the co-electrolysis device 30 is controlled by the control unit 60. As a result, the efficiency of CH₄ produced in the reforming device 40 is optimized.

The co-electrolysis device 30 includes a manifold 31 and a plurality of co-electrolysis cells 32.

The manifold 31 has a configuration in which COz and H₂O can be distributed to the co-electrolysis cells 32 and H₂ and CO can be collected from the co-electrolysis device 30. The manifold 31 internally has a gas supply chamber 31a and a gas collection chamber 31b. The gas supply chamber 31a and the gas collection chamber 31b are airtightly separated from each other. The first pipe L1 is connected to the gas supply chamber 31a. COz and H₂O are supplied from the first pipe L1 to the gas supply chamber 31a. The gas collection chamber 31b collects the H₂ and CO produced in the co-electrolysis cells 32. A second pipe L2 is connected to the gas collection chamber 31b. H₂ and CO are discharged from the gas collection chamber 31b to the second pipe L2.

Abase end portion of each co-electrolysis cell 32 is supported by the manifold 31. A leading end portion of each co-electrolysis cell 32 is a free end. CO₂ and H₂O are supplied from the gas supply chamber 31a to each co-electrolysis cell 32. In each co-electrolysis cell 32, H₂, CO, and O²⁻ (oxygen ions) are produced from COz and H₂O at the later-described hydrogen electrode 2, and Oz is produced from O²⁻ at the later-described oxygen electrode 4. The produced H₂ and CO are collected into the gas collection chamber 31b and discharged from the second pipe L2. Note that the number of co-electrolysis cells 32 is not particularly limited as long as it is 1 or more.

Each co-electrolysis cell 32 produces H₂ (hydrogen), CO, and Oz (oxygen) by co-electrolyzing COz and HzO. The term "co-electrolysis" used in this specification refers to production of H₂, CO, and Oz by electrolyzing COz and H₂O together. H₂ and CO are collected in the co-electrolysis device 30, and Oz is released to the outside of the co-electrolysis device 30. Each co-electrolysis cell 32 operates at high temperatures (e.g., 600°C to 850°C). The operating temperatures of the co-electrolysis cells 32 are determined in consideration of the performance of the electrolyte 3 and the durability of the co-electrolysis cells 32. The wording "operating temperature of the co-electrolysis cell 32" used in this specification refers to the temperature at the center in the longitudinal direction (X-axis direction) of the co-electrolysis cell 32.

An example of a configuration of the co-electrolysis device 30 will be described later.

The reforming device 40 is connected to the co-electrolysis device 30. In this embodiment, the reforming device 40 is directly connected to the co-electrolysis device 30 by the second pipe L2. Therefore, the H₂ and CO produced in the co-electrolysis device 30 are directly supplied to the reforming device 40 without changing their compositions.

The reforming device 40 has a manifold 41 and a plurality of reforming cells 42.

The manifold 41 has a configuration in which gas can be distributed to the reforming cells 42 and gas can be collected from the reforming cells 42. The manifold 41 internally has a gas supply chamber 41a and a gas collection chamber 41b. The gas supply chamber 41a and the gas collection chamber 41b are airtightly separated from each other. The gas supply chamber 41a is connected to the second pipe L2. H₂ and CO are supplied from the second pipe L2 to the gas supply chamber 41a. The gas collection chamber 41b collects the CH₄ and H₂O produced in the reforming cells 42. The gas collection chamber 41b is connected to the third pipe L3. A reforming catalyst may be placed in the gas collection chamber 41b. The reforming catalyst may be in the form of pellets. The gas collection chamber 41b may be filled with the reforming catalyst. It is possible to use Ru/Al₂O₃, Ni/Al₂O₃, or the like as a reforming catalyst, for example.

A base end portion of each reforming cell 42 is supported by the manifold 41. A leading end portion of each reforming cell 42 is a free end. H₂ and CO are supplied from the gas supply chamber 41a to the reforming cells 42. In the reforming cells 42, CH₄ and H₂O are produced from H₂ and CO by the later-described reforming catalyst. The produced CH₄ and H₂O are collected into the gas collection chamber 41b and discharged from the third pipe L3 to the outside of the methane production system 1. Note that the number of reforming cells 42 is not particularly limited as long as it is 1 or more.

In the reforming cells 42, CH₄ (methane) and H₂O are produced from the H₂ and CO produced in the co-electrolysis device 30. The term "reforming" used in this specification refers to production of CH₄ and H₂O from H₂ and CO. Each reforming cell 42 operates at a temperature (e.g., 200°C to 500°C) lower than the operating temperature of the co-electrolysis cell 32. The wording "operating temperature of the reforming cell 42" used in this specification refers to the temperature at the center in the longitudinal direction (X-axis direction) of the reforming cell 42. When the later-described support substrate 45 of the reforming cell 42 contains MgO (magnesium oxide), if the operating temperature is lower than 300°C, MgCO₃ (magnesium carbonate) will precipitate in the support substrate 45. Thus, the operating temperature of the reforming cell 42 is preferably 350°C or more.

The volume of a space in which the reforming cells 42 are installed in the reforming device 40 (referred to as "reforming cell installation volume" hereinafter) is preferably smaller than the volume of a space where the co-electrolysis cells 32 are installed in the co-electrolysis device 30 (referred to as "co-electrolysis cell installation volume" hereinafter). As a result, it is possible to increase the heat generation density of the reforming cells 42 where exothermic methane production reaction occurs, and to easily control the temperature of the reforming cells 42.

Note that the reforming cell installation volume is the sum of the volume of gaps between opposing reforming cells 42 and the volume of the reforming cells 42. Similarly, the co-electrolysis cell installation volume is the sum of the volume of gaps between opposing co-electrolysis cells 32 and the volume of the co-electrolysis cells 32.

An example of a configuration of the reforming device 40 will be described later.

The voltage detector 50 detects an electromotive voltage of at least one of the plurality of reforming cells 42. In this embodiment, the voltage detector 50 detects the electromotive voltage of the most downstream reforming cell 42 (hereinafter referred to as "most-downstream reforming cell 42") closest to the inlet of the third pipe L3 (an opening in the gas collection chamber 41b), out of the plurality of reforming cells 42. The electromotive voltage of the most-downstream reforming cell 42 is an index indicating the efficiency of CH₄ produced in the reforming device 40. When the electromotive voltage of the most-downstream reforming cell 42 is smaller than a predetermined value, it means that the efficiency of CH₄ produced in the reforming device 40 is not sufficient. However, the voltage detector 50 may detect the electromotive voltages of reforming cells 42 other than the most-downstream reforming cell 42.

The control unit 60 controls the amount of H₂O supplied from the H₂O supply device 20 to the co-electrolysis device 30. The control unit 60 changes the amount of H₂O supplied, according to the electromotive voltage of the most-downstream reforming cell 42 detected by the voltage detector 50. Specifically, if the electromotive voltage of the most-downstream reforming cell 42 is smaller than a predetermined value, the control unit 60 determines that the efficiency of CH₄ produced in the reforming device 40 is not sufficient, and reduces the amount of H₂O supplied from the H₂O supply device 20 to the co-electrolysis device 30. Accordingly, the efficiency of CH₄ produced in the reforming device 40 is improved and the electromotive voltage of the most-downstream reforming cell 42 is increased. As a result, the efficiency of CH₄ produced in the reforming device 40 is maintained in a desired appropriate range.

### Configuration of co-electrolysis device 30

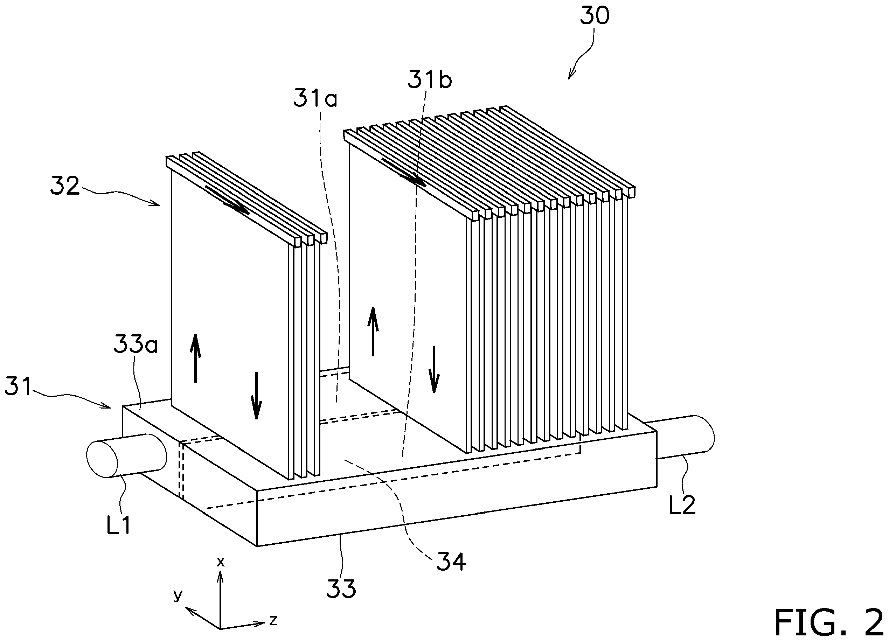

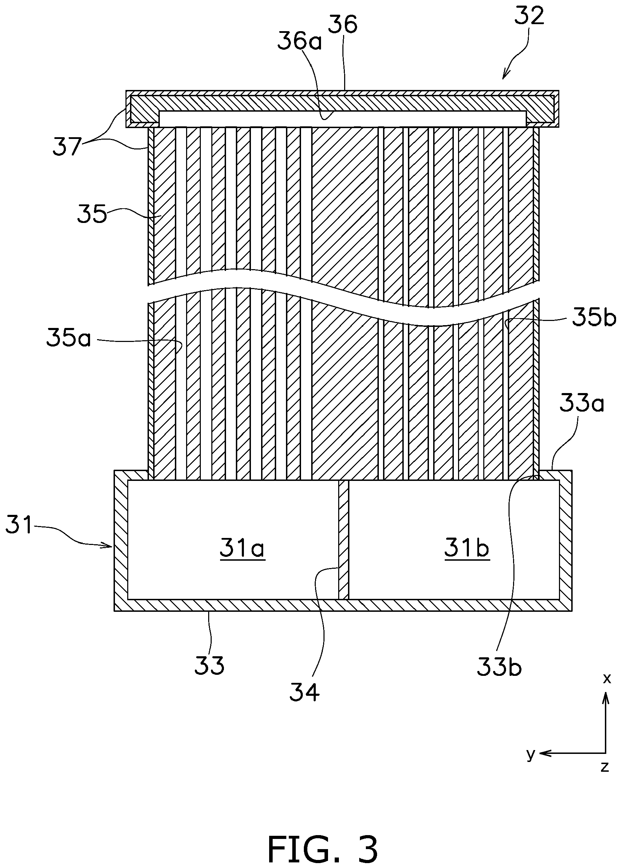

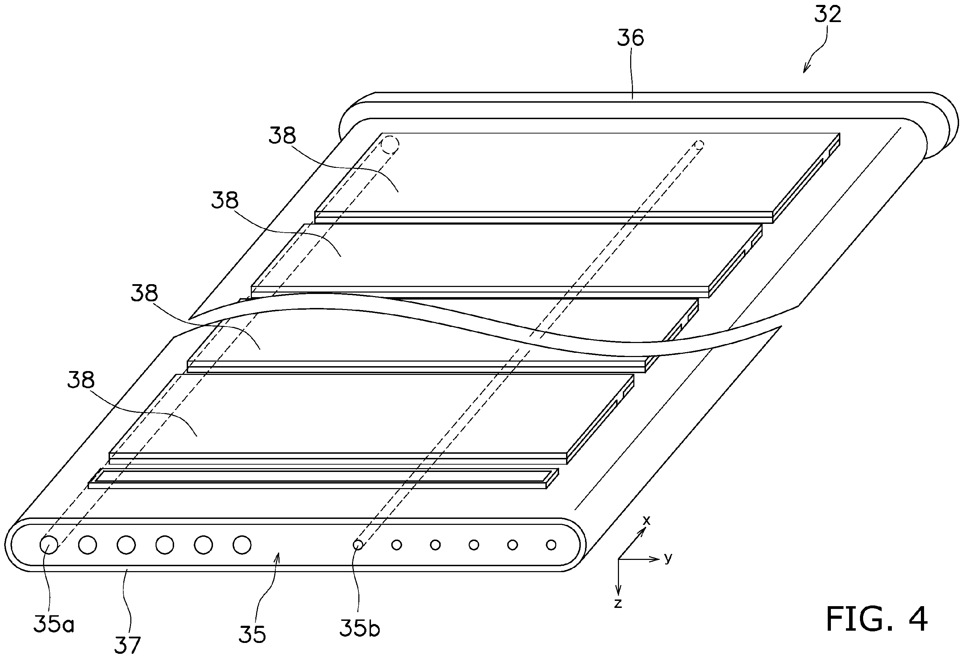

FIG. 2 is a perspective view of the co-electrolysis device 30. FIG. 3 is a cross-sectional view of the co-electrolysis device 30. FIG. 4 is a perspective view of the co-electrolysis cell 32. Some of the co-electrolysis cells 32 are not shown in FIG. 2.

### Manifold 31

As shown in FIGS. 2 and 3, the manifold 31 includes a manifold main body portion 33 and a partition plate 34.

The manifold main body portion 33 is hollow. The partition plate 34 is arranged in the manifold main body portion 33. The partition plate 34 airtightly separates the gas supply chamber 31a and the gas collection chamber 31b from each other.

The manifold main body portion 33 has a top plate portion 33a. As shown in FIG. 3, the top plate portion 33a is provided with a plurality of through holes 33b. The through holes 33b are arranged side-by-side at predetermined intervals in the longitudinal direction (Z-axis direction) of the manifold main body portion 33. Each through hole 33b extends in the width direction (Y-axis direction) of the manifold main body portion 33. Although each through hole 33b is a long hole that is in communication with the gas supply chamber 31a and the gas collection chamber 31b in this embodiment, the through hole 33b may be divided into a hole that is in communication with the gas supply chamber 31a and a hole that is in communication with the gas collection chamber 31b.

### Co-electrolysis cell 32

As shown in FIGS. 2 and 3, each co-electrolysis cell 32 extends in a direction away from the manifold 31. Abase end portion of each co-electrolysis cell 32 is fixed to the through hole 33b of the top plate portion 33a using a bonding material (not shown) or the like. The base end portion of the co-electrolysis cell 32 may be inserted into the through hole 33b, or may protrude outward of the through hole 33b.

The co-electrolysis cells 32 are disposed such that their main surfaces face each other. The co-electrolysis cells 32 are arranged side-by-side at predetermined intervals along the longitudinal direction (Z-axis direction) of the manifold 31. That is, the arrangement direction of the co-electrolysis cells 32 extends in the longitudinal direction of the manifold 31. The co-electrolysis cells 32 are electrically connected in series or in a combination of series and parallel connections, using current collector members (not shown).

As shown in FIG. 3 and 4, each co-electrolysis cell 32 includes a support substrate 35, a connection member 36, a coating layer 37, and a plurality of element portions 38. The co-electrolysis cell 32 according to this embodiment is a so-called horizontal-stripe type solid oxide electrolysis cell (SOEC).

The support substrate 35 is plate-shaped. In this embodiment, the vertical direction (X-axis direction) in FIG. 3 is the longitudinal direction of the support substrate 35, and the horizontal direction (Y-axis direction) in FIG. 3 is the width direction of the support substrate 35.

A plurality of first gas channels 35a and a plurality of second gas channels 35b are formed in the support substrate 35. The first gas channels 35a and the second gas channels 35b each extend from the base end portion to the leading end portion of the co-electrolysis cell 32 in the support substrate 35. The first gas channels 35a and the second gas channels 35b pass through the support substrate 35. The first gas channels 35a are disposed at intervals in the width direction of the support substrate 35. The second gas channels 35b are disposed at intervals in the width direction of the support substrate 35. Although the inner diameter of the first gas channels 35a is larger than the inner diameter of the second gas channels 35b in this embodiment, the inner diameter of the first gas channels 35a and the inner diameter of the second gas channels 35b are not particularly limited.

The first gas channels 35a are open to the gas supply chamber 31a. COz and H₂O flow into the first gas channels 35a from the gas supply chamber 31a. The second gas channels 35b are open to the gas collection chamber 31b. H₂ and CO flow out from the second gas channels 35b and enter the gas collection chamber 31b.

The support substrate 35 is made of a porous material having no electron conductivity so as to allow gas permeation while preventing short circuits between element portions 38. The support substrate 35 may be made of CSZ (calcia-stabilized zirconia), 8YSZ (yttria-stabilized zirconia), Y₂O₃ (yttria), MgO (magnesium oxide), MgAl₂O₄ (magnesia alumina spinel), or a composite thereof, for example. The support substrate 35 may have a porosity of 20% to 60%. Note that the porosity mentioned in this specification is a value measured using the Archimedes' method.

The connection member 36 is attached to the leading end portion of the support substrate 35. The connection member 36 may be made of a porous material similar to that of the support substrate 35, for example. The connection member 36 internally has a connection channel 36a. The connection channel 36a is in communication with the first gas channels 35a and the second gas channels 35b.

The coating layer 37 covers outer surfaces of the support substrate 35 and the connection member 36. The coating layer 37 is denser than the support substrate 35 and the connection member 36. The coating layer 37 may have a porosity of about 0% to 7%. The coating layer 37 may be made of a material used in the later-described electrolyte 3, crystallized glass, or the like.

The element portions 38 are supported by the support substrate 35. The element portions 38 may be arranged on both main surfaces of the support substrate 35, or may be arranged on only one of the main surfaces.

### Element portion 38

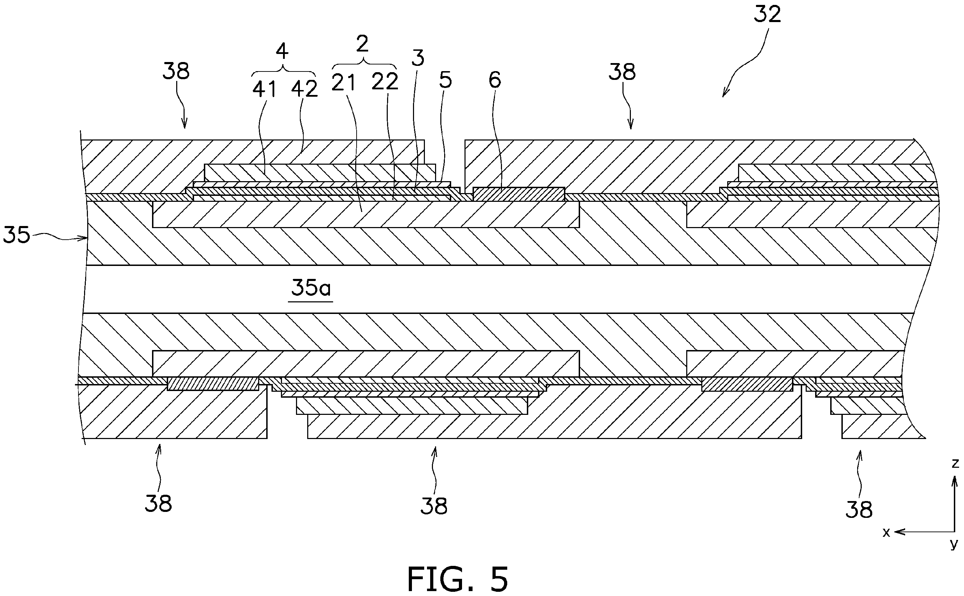

FIG. 5 is a cross-sectional view of the co-electrolysis cell 32 cut along the first gas channel 35a.

Each element portion 38 has a hydrogen electrode 2, an electrolyte 3, an oxygen electrode 4, a reaction preventing film 5, and an interconnector 6. The hydrogen electrode 2 is an example of a "first electrode" according to the present invention. The electrolyte 3 is an example of an "electrolyte" according to the present invention. The oxygen electrode 4 is an example of a "second electrode" according to the present invention.

At the hydrogen electrode 2, H₂, CO, and O²⁻ are produced from COz and H₂O according to the chemical reaction of the co-electrolysis indicated by Chemical Equation (1) below.
- Hydrogen electrode 2:

   COz + H₂O + 4e⁻ → CO + H₂ + 2O²⁻ ••• (1)

The hydrogen electrode 2 has a hydrogen electrode base body 21 and a hydrogen electrode active portion 22.

The hydrogen electrode base body 21 is disposed on the support substrate 35. The hydrogen electrode base body 21 is embedded in a recess formed in a surface of the support substrate 35 in this embodiment, but may be placed on the surface of the support substrate 35. The hydrogen electrode base body 21 may have a thickness of 50 to 500 µm.

The hydrogen electrode base body 21 is made of a porous material having electron conductivity. The hydrogen electrode base body 21 preferably has higher electron conductivity than the hydrogen electrode active portion 22. The hydrogen electrode base body 21 optionally has oxygen ion conductivity. The hydrogen electrode base body 21 may be made of a composite of NiO and 8YSZ, a composite of NiO and Y₂O₃, a composite of NiO and CSZ, or the like, for example.

The hydrogen electrode active portion 22 is disposed on the hydrogen electrode base body 21. The hydrogen electrode active portion 22 may have a thickness of 5 to 100 µm. The hydrogen electrode active portion 22 has oxygen ion conductivity and electron conductivity. The hydrogen electrode active portion 22 preferably has higher oxygen ion conductivity than the hydrogen electrode base body 21. The hydrogen electrode active portion 22 may be made of a composite of NiO and 8YSZ, a composite of NiO and GDC (Ce, Gd)Oz (gadolinium doped ceria), or the like, for example.

The electrolyte 3 is disposed between the hydrogen electrode 2 and the oxygen electrode 4. The electrolyte 3 transfers O²⁻ produced at the hydrogen electrode 2 to the oxygen electrode 4. The electrolyte 3 is disposed on the hydrogen electrode 2. In this embodiment, the electrolyte 3 extends in the longitudinal direction of the support substrate 35 between two interconnectors 6. The electrolyte 3 may have a thickness of 3 to 50 µm, for example.

The electrolyte 3 is made of a dense material that have oxygen ion conductivity and does not have electron conductivity. The electrolyte 3 is denser than the support substrate 35. The electrolyte 3 may have a porosity of 0% to 7%, for example. The electrolyte 3 may be made of 8YSZ, LSGM (lanthanum gallate), or the like, for example.

At the oxygen electrode 4, Oz is produced from O²⁻ transferred from the hydrogen electrode 2 through the electrolyte 3, according to the chemical reaction indicated by Chemical Equation (2) below.
- Oxygen electrode 4:

   2O²⁻ → O₂ + 4e⁻ ••• (2)

The oxygen electrode 4 has an oxygen electrode active portion 41 and an oxygen electrode base body 42.

The oxygen electrode active portion 41 is disposed on the reaction preventing film 5. The oxygen electrode active portion 41 may have a thickness of 10 to 100 µm, for example.

The oxygen electrode active portion 41 is made of a porous material having oxygen ion conductivity and electron conductivity. The oxygen electrode active portion 41 preferably has higher oxygen ion conductivity than the oxygen electrode base body 42. The oxygen electrode active portion 41 may be made of LSCF=(La, Sr)(Co, Fe)Os (lanthanum strontium cobalt ferrite), LSF=(La, Sr) FeO₃ (lanthanum strontium ferrite), LNF=La(Ni, Fe)O₃ (lanthanum nickel ferrite), LSC=(La, Sr)CoO₃ (lanthanum strontium cobaltite), SSC=(Sm, Sr)CoOs (samarium strontium cobaltite), or the like, for example.

The oxygen electrode base body 42 is disposed on the oxygen electrode active portion 41. The oxygen electrode base body 42 is electrically connected to the hydrogen electrode base body 21 of the adjacent element portion 38 via the interconnector 6. The oxygen electrode base body 42 may have a thickness of 50 to 500 µm, for example.

The oxygen electrode base body 42 is made of a porous material having electron conductivity. The oxygen electrode base body 42 preferably has higher electron conductivity than the oxygen electrode active portion 41. The oxygen electrode base body 42 optionally has oxygen ion conductivity. The oxygen electrode base body 42 may be made of LSCF, LSC, Ag (silver), Ag-Pd (silver palladium alloy), or the like, for example.

The reaction preventing film 5 is disposed between the electrolyte 3 and the oxygen electrode active portion 41. The reaction preventing film 5 suppresses a reaction of substances contained in the electrolyte 3 and the oxygen electrode active portion 41 to form a reaction layer having high electric resistance. The reaction preventing film 5 may have a thickness of 3 to 50 µm, for example. The reaction preventing film 5 is made of a dense material. The reaction preventing film 5 may be made of GDC, for example.

The interconnector 6 is connected to the oxygen electrode base body 42 and connected to the hydrogen electrode base body 21 of the adjacent element portion 38. The interconnector 6 may have a thickness of 10 to 100 µm, for example. The interconnector 6 is made of a dense material that have electron conductivity. The interconnector 6 is denser than the support substrate 35. The interconnector 6 may have a porosity of 0% to 7%. The interconnector 6 may be made of LaCrC₃ (lanthanum chromite), (Sr, La)TiO₃ (strontium titanate), or the like, for example.

### Configuration of reforming device 40

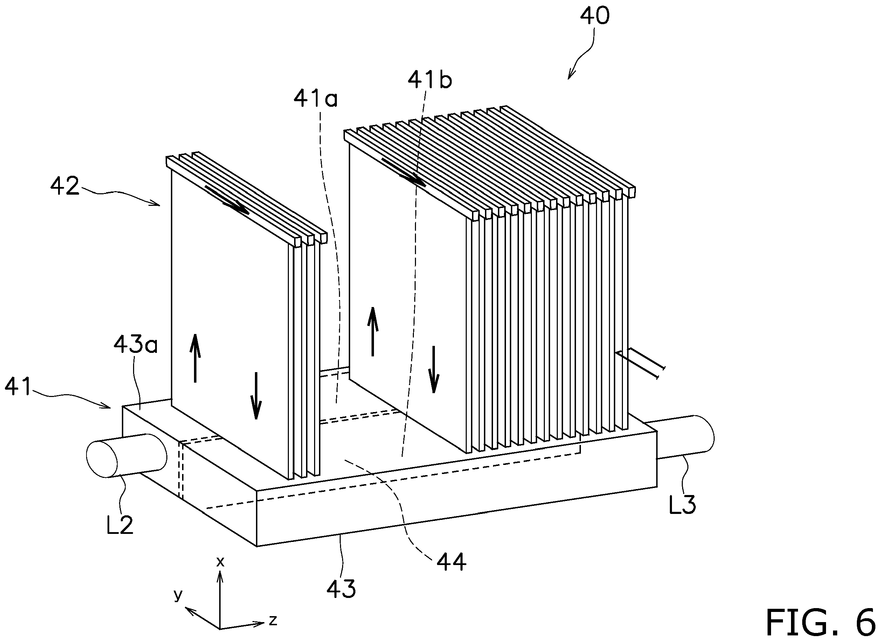

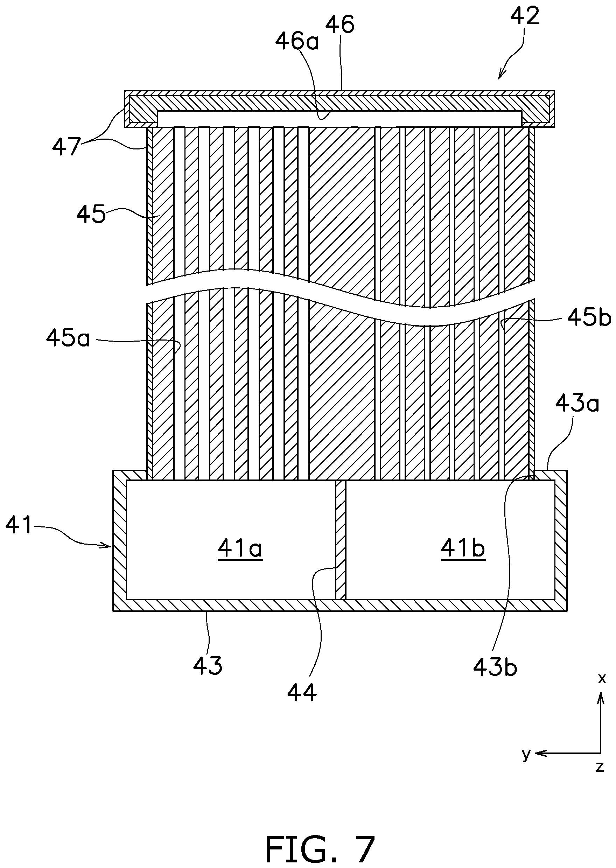

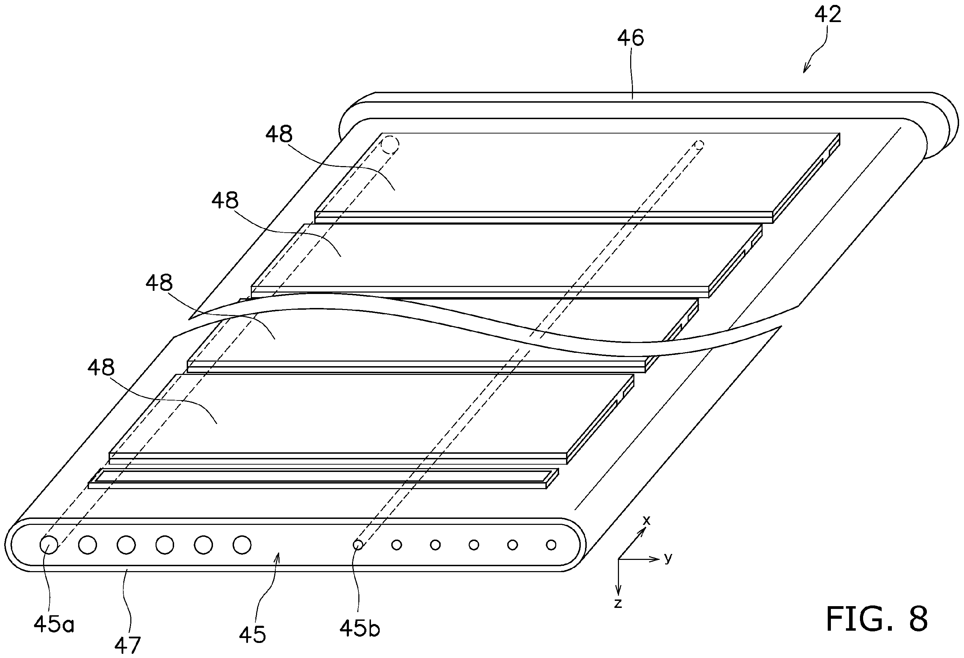

FIG. 6 is a perspective view of the reforming device 40. FIG. 7 is a cross-sectional view of the reforming device 40. FIG. 8 is a perspective view of the reforming device 42. Some of the reforming cells 42 are not shown in FIG. 6.

### Manifold 41

As shown in FIGS. 6 and 7, the manifold 41 includes a manifold main body portion 43 and a partition plate 44.

The manifold main body portion 43 is hollow. The partition plate 44 is arranged in the manifold main body portion 43. The partition plate 44 airtightly separates the gas supply chamber 41a and the gas collection chamber 41b from each other.

The manifold main body portion 43 has a top plate portion 43a. As shown in FIG. 7, the top plate portion 43a is provided with a plurality of through holes 43b. The through holes 43b are arranged side-by-side at predetermined intervals in the longitudinal direction (Z-axis direction) of the manifold main body portion 43. Each through hole 43b extends in the width direction (Y-axis direction) of the manifold main body portion 43. Although each through hole 43b is a long hole that is in communication with the gas supply chamber 41a and the gas collection chamber 41b in this embodiment, the through hole 43b may be divided into a hole that is in communication with the gas supply chamber 41a and a hole that is in communication with the gas collection chamber 41b.

### Reforming cell 42

As shown in FIGS. 6 and 7, each reforming cell 42 extends in a direction away from the manifold 41. A base end portion of each reforming cell 42 is fixed to the through hole 43b of the top plate portion 43a using a bonding material (not shown) or the like. The base end portion of the reforming cell 42 may be inserted into the through hole 43b, or may protrude outward of the through hole 43b.

The reforming cells 42 are disposed such that their main surfaces face each other. The reforming cells 42 are arranged side-by-side at predetermined intervals along the longitudinal direction (Z-axis direction) of the manifold 41. That is, the arrangement direction of the reforming cells 42 is the same as the longitudinal direction of the manifold 41. The reforming cells 42 are electrically connected in series by current collector members (not shown). In this embodiment, the voltage detector 50 is connected to the most-downstream reforming cell 42 out of the plurality of reforming cells 42.

As shown in FIGS. 7 and 8, each reforming cell 42 includes a support substrate 45, a connection member 46, a coating layer 47, and a plurality of reforming element portions 48. The reforming cell 42 according to this embodiment has the same configuration as the above-described co-electrolysis cell 32. The wording "same configuration" used in this specification indicates that constituent members have substantially the same shape and materials of the constituent members are substantially the same, and it does not matter whether defects or impairments occur during or after production.

The support substrate 45 is plate-shaped. In this embodiment, the vertical direction (X-axis direction) in FIG. 7 is the longitudinal direction of the support substrate 45, and the horizontal direction (Y-axis direction) in FIG. 7 is the width direction of the support substrate 45.

A plurality of first gas channels 45a and a plurality of second gas channels 45b are formed in the support substrate 45. The first gas channels 45a and the second gas channels 45b each extend from the base end portion to the leading end portion of the reforming cell 42 in the support substrate 45. The first gas channels 45a and the second gas channels 45b pass through the support substrate 45. The first gas channels 45a are disposed at intervals in the width direction of the support substrate 45. The second gas channels 45b are disposed at intervals in the width direction of the support substrate 45. Although the inner diameter of the first gas channels 45a is larger than the inner diameter of the second gas channels 45b in this embodiment, the inner diameter of the first gas channels 45a and the inner diameter of the second gas channels 45b are not particularly limited.

The first gas channels 45a are open to the gas supply chamber 41a. COz and H₂O flow into the first gas channels 45a from the gas supply chamber 41a. The second gas channels 45b are open to the gas collection chamber 41b. H₂ and CO flow out from the second gas channels 45b and enter the gas collection chamber 41b.

The support substrate 45 is made of a porous material having no electron conductivity so as to allow gas permeation while preventing short circuits between reforming element portions 48. The support substrate 45 may be made of a composite of CSZ, NiO, and 8YSZ, a composite of NiO and Y₂O₃, a composite of MgO and MgAl₂O₄, or the like, for example. The support substrate 45 may have a porosity of 20% to 60%.

The connection member 46 is attached to the leading end portion of the support substrate 45. The connection member 46 may be made of a porous material similar to that of the support substrate 45, for example. The connection member 46 internally has a connection channel 46a. The connection channel 46a is in communication with the first gas channels 45a and the second gas channels 45b.

The coating layer 47 covers outer surfaces of the support substrate 45 and the connection member 46. The coating layer 47 is denser than the support substrate 45 and the connection member 46. The coating layer 47 may have a porosity of about 0% to 7%. The coating layer 47 may be made of a material used in the later-described dense film 3a, crystallized glass, or the like.

The reforming element portions 48 are supported by the support substrate 45. The reforming element portions 48 may be arranged on both main surfaces of the support substrate 45, or may be arranged on only one of the main surfaces.

### Reforming element portion 48

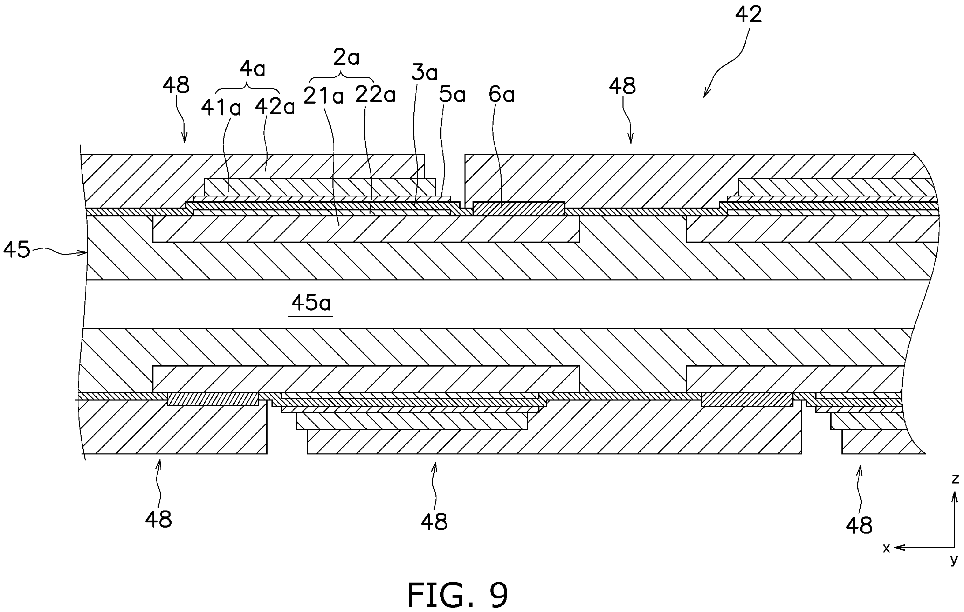

FIG. 9 is a cross-sectional view of the reforming cell 42 cut along the first gas channel 45a.

Each reforming element portion 48 includes a first electrode 2a, a dense film 3a, a second electrode 4a, a reaction preventing film 5a, and an interconnector 6a. The reforming element portion 48 according to this embodiment has the same configuration as the above-described element portion 38 of the co-electrolysis cell 32.

At the first electrode 2a, CH₄ and H₂O are produced from the H₂ and CO produced at the hydrogen electrode 2 of the co-electrolysis cell 32, according to the chemical reaction indicated by Chemical Equation (3) below.
- First electrode 2a:

   3H₂ + CO → CH₄ + H₂O • • • (3)

The first electrode 2a has a first electrode base body 21a and a first electrode catalyst portion 22a.

The first electrode base body 21a is disposed on the support substrate 45. The first electrode base body 21a is embedded in a recess formed in a surface of the support substrate 45 in this embodiment, but may be placed on the surface of the support substrate 45. The first electrode base body 21a may have a thickness of 50 to 500 µm.

The first electrode base body 21a is made of a porous material having electron conductivity. The first electrode base body 21a need not have oxygen ion conductivity. The first electrode base body 21a may be made of a composite of NiO and 8YSZ, a composite of NiO and Y₂O₃, a composite of NiO and CSZ, or the like, for example.

The first electrode catalyst portion 22a is disposed on the first electrode base body 21a. The first electrode catalyst portion 22a may have a thickness of 5 to 30 µm. The first electrode catalyst portion 22a contains a reforming catalyst for producing CH₄ and H₂O from H₂ and CO. The first electrode catalyst portion 22a has electron conductivity. The first electrode catalyst portion 22a need not have oxygen ion conductivity. The first electrode catalyst portion 22a may be made of a composite of NiO and 8YSZ, a composite of NiO and GDC, or the like, for example. In this case, Ni functions as a reforming catalyst for producing CH₄ and H₂O from H₂ and CO.

The dense film 3a is disposed on the first electrode 2a. In this embodiment, the dense film 3a extends in the longitudinal direction of the support substrate 45 between two interconnectors 6a. The dense film 3a may have a thickness of 3 to 50 µm, for example.

The dense film 3a is made of a dense material. The dense film 3a is denser than the support substrate 45. The dense film 3a may have a porosity of 0% to 7%, for example. The dense film 3a need not have oxygen ion conductivity or electron conductivity. The dense film 3a may be made of 8YSZ, LSGM (lanthanum gallate), or the like, for example.

The second electrode 4a has a second electrode active portion 41a and a second electrode base body 42a.

The second electrode active portion 41a is disposed on the reaction preventing film 5a. The second electrode active portion 41a may have a thickness of 10 to 100 µm, for example.

The second electrode active portion 41a is made of a porous material having electron conductivity. The second electrode active portion 41a need not have oxygen ion conductivity. The second electrode active portion 41a may be made of LSCF, LSF, LNF, LSC, SSC, or the like, for example.

The second electrode base body 42a is disposed on the second electrode active portion 41a. The second electrode base body 42a is electrically connected to the first electrode base body 21a of the adjacent reforming element portion 48 via the interconnector 6a. The second electrode base body 42a may have a thickness of 50 to 500 µm, for example.

The second electrode base body 42a is made of a porous material having electron conductivity. The second electrode base body 42a need not have oxygen ion conductivity. The second electrode base body 42a may be made of LSCF, LSC, Ag (silver), Ag-Pd (silver palladium alloy), or the like, for example.

The reaction preventing film 5a is disposed between the dense film 3a and the second electrode active portion 41a. The reaction preventing film 5a suppresses a reaction of substances contained in the dense film 3a and the second electrode active portion 41a to form a reaction layer having high electric resistance. The reaction preventing film 5a may have a thickness of 3 to 50 µm, for example. The reaction preventing film 5a is made of a dense material. The reaction preventing film 5a may be made of GDC, for example.

The interconnector 6a is connected to the second electrode base body 42a and the first electrode base body 21a of the adjacent reforming element portion 48. The interconnector 6a may have a thickness of 10 to 100 µm, for example. The interconnector 6a is made of a dense material that have electron conductivity. The interconnector 6a is denser than the support substrate 45. The interconnector 6a may have a porosity of 0% to 7%. The interconnector 6a may be made of LaCrO₃ (lanthanum chromite), (Sr, La)TiO₃ (strontium titanate), or the like, for example.

### Features

(1) The methane production system 1 includes the co-electrolysis device 30 and the reforming device 40 connected to the co-electrolysis device 30. The co-electrolysis device 30 has the co-electrolysis cells 32 that include the hydrogen electrodes 2 at which H₂, CO, and O²⁻ are produced from COz and HzO, and the electrolytes 3 capable of transferring O²⁻, and the oxygen electrodes 4 at which Oz is produced from O²⁻ transferred from the hydrogen electrodes 2 through the electrolytes 3. The reforming device 40 has the reforming cells 42 that produce CH₄ from the H₂ and CO produced at the hydrogen electrodes 2.
   Thus, CH₄ can be produced on-site using the H₂ and CO produced in the co-electrolysis device 30. Therefore, H₂ and CO do not need to be transported from a plant where an SOEC is installed to a plant where a reforming device is installed.
(2) The most-downstream reforming cell 42 includes the first electrodes 2a containing a reforming catalyst, the second electrodes 4a, and the dense films 3a each disposed between the first electrode 2a and the second electrode 4a. The control unit 60 changes the amount of H₂O supplied, according to the electromotive voltage of the most-downstream reforming cell 42 detected by the voltage detector 50. As a result, the efficiency of CH₄ produced in the reforming device 40 can be maintained in a desired appropriate range.
(3) The reforming cell 40 has the same configuration as the co-electrolysis cell 32. As a result, the co-electrolysis cell 32 and the reforming cell 40 do not need to be produced separately, and thus a production cost can be reduced.

### Variation of embodiment

Although an embodiment of the present invention has been described above, the present invention is not limited thereto, and various modifications can be made without departing from the spirit of the present invention.

### Variation 1

Although the co-electrolysis cell 32 is a horizontal-stripe type SOEC in the above embodiment, the co-electrolysis cell 32 is not limited to this. The co-electrolysis cell 32 may be a vertical-stripe type (hollow flat plate type), flat plate type, or cylindrical type SOEC, or the like. A configuration of a vertical-stripe type SOEC is described in JP 2015-125897A, for example. A configuration of a flat plate type SOEC is described in JP 2020-177839A, for example. A configuration of a cylindrical type SOEC is described in JP 2008-270203A, for example. However, horizontal-stripe type SOECs are particularly preferable because they have higher H₂O utilization efficiency than other SOECs.

### Variation 2

Although each element portion 38 has the hydrogen electrode 2, the electrolyte 3, the oxygen electrode 4, the reaction preventing film 5, and the interconnector 6 in the above embodiment, it is sufficient that the element portion 38 includes at least the hydrogen electrode 2, the electrolyte 3, and the oxygen electrode 4.

### Variation 3

Although the reforming cells 42 has the same configuration as the co-electrolysis cells 32 in the above embodiment, the reforming cells 42 may have a configuration different from that of the co-electrolysis cells 32.

### Variation 4

Although the voltage detector 50 detects the electromotive voltage of the most-downstream reforming cell 42 in the above embodiment, the electromotive voltages of the reforming cells 42 other than the most-downstream reforming cell 42 may be detected, or the average electromotive voltage of a plurality of reforming cells 42 may be detected.

### Variation 5

Although the control unit 60 changes the amount of H₂O supplied, according to the electromotive voltage of the most-downstream reforming cell 42 detected by the voltage detector 50 in the above embodiment, the amount of H₂O supplied may be kept at a constant value. In this case, the reforming cell 42 does not need to have the first and second electrodes 2a and 4a, and it is sufficient that the reforming cell 42 has a reforming catalyst that comes into contact with H₂ and CO. The reforming catalyst may be placed in the dense film 3a.

### Variation 6

Although the entirety of gas discharged from the reforming device 40 (CH₄ and H₂O produced in the reforming cells 42) is discharged from the third pipe L3 to the outside of the methane production system 1 in the above embodiment, the present invention is not limited to this.

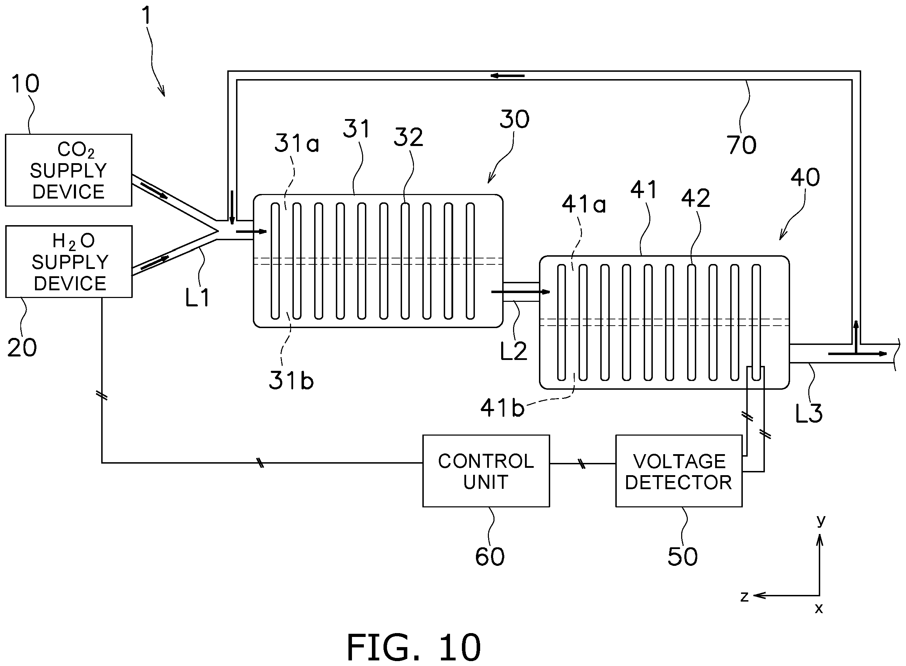

As shown in FIG. 10, the methane production system 1 may further include a reflux portion 70 that refluxes part of gas discharged from the reforming device 40 to the co-electrolysis device 30, for example. The reflux portion 70 is a pipe that is in communication with the first pipe L1 and the third pipe L3. By refluxing part of gas discharged from the reforming device 40 to the co-electrolysis device 30 in this manner, part of CO or H₂ contained in the discharged gas can be supplied to the hydrogen electrode 2 of the co-electrolysis device 30. Thus, the efficiency of electrolysis can be increased by facilitating vaporization of water at the hydrogen electrode 2. Also, if the hydrogen electrode active portion 22 of the hydrogen electrode 2 in the co-electrolysis device 30 contains Ni, the oxidation of Ni can be suppressed due to reducing action of CO or H₂.

Note that the reflux amount of discharged gas can be easily controlled by adjusting the inner diameter of a pipe that constitutes the reflux portion 70.

### REFERENCE SIGNS LIST

- 1: Methane production system
- 10: COz supply device
- 20: H₂O supply device
- 30: Co-electrolysis device
- 31: Manifold
- 32: Co-electrolysis cell
- 38: Element portion
- 2: Hydrogen electrode
- 3: Electrolyte
- 4: Oxygen electrode
- 40: Reforming device
- 41: Manifold
- 42: Reforming cell
- 48: Reforming element portion
- 2a: First electrode
- 3a: Dense film
- 4a: Second electrode
- 50: Voltage detector
- 60: Control unit
- 70: Reflux portion
- L1: First pipe
- L2: Second pipe
- L3: Third pipe

## Claims

1. A methane production system comprising:
a co-electrolysis device; and
a reforming device connected to the co-electrolysis device, wherein
the co-electrolysis device has a co-electrolysis cell including a first electrode at which H₂, CO, and O²⁻ are produced from COz and HzO, an electrolyte capable of transferring O²⁻, and a second electrode at which Oz is produced from the O²⁻ transferred from the first electrode through the electrolyte, and
the reforming device has a reforming cell configured to produce CH₄ from the H₂ and CO produced at the first electrode.

2. The methane production system according to claim 1, wherein
the reforming cell contains a reforming catalyst for producing CH₄ from the H₂ and CO produced at the first electrode.

3. The methane production system according to claim 2, wherein
the reforming cell includes a first electrode containing the reforming catalyst, a second electrode, and a dense film disposed between the first electrode and the second electrode.

4. The methane production system according to claim 3, further comprising:
a H₂O supply device configured to supply H₂O to the co-electrolysis device;
a control unit configured to control an amount of H₂O supplied from the H₂O supply device to the co-electrolysis device; and
a voltage detector configured to detect an electromotive voltage of the reforming cell, wherein
the control unit changes the amount of H₂O supplied, according to the electromotive voltage of the reforming cell detected by the voltage detector.

5. The methane production system according to claim 3 or 4, wherein
the reforming cell has the same configuration as the co-electrolysis cell.

6. The methane production system according to any one of claims 1 to 5, wherein
an operating temperature of the reforming cell is lower than an operating temperature of the co-electrolysis cell.

7. The methane production system according to any one of claims 1 to 6, further comprising
a reflux portion configured to reflux part of gas discharged from the reforming device to the co-electrolysis device.
